(19) Europäisches Patentamt
European Patent Office
Office européen des brevets

(11) **EP 0 207 806 B1**

## (12) EUROPEAN PATENT SPECIFICATION

(45) Date of publication and mention
of the grant of the patent:
**19.06.1996 Bulletin 1996/25**

(51) Int. Cl.$^6$: **A61B 5/02**, G06F 17/00

(21) Application number: **86305199.1**

(22) Date of filing: **04.07.1986**

(54) **Improved automated systolic blood pressure monitor with data enhancement**

Gerät zur Überwachung des systolischen Blutdrucks mit Daten-Aufbereitung

Appareil de surveillance de la pression systolique comportant une mémoire de rafraîchissement de données

(84) Designated Contracting States:
**AT BE CH DE FR GB IT LI LU NL SE**

(30) Priority: **05.07.1985 US 751827**

(43) Date of publication of application:
**07.01.1987 Bulletin 1987/02**

(73) Proprietor: **CRITIKON, INC.**
**Tampa Florida 33607 (US)**

(72) Inventors:
• **Ramsey III, Maynard**
**Tampa, FL 33609 (US)**
• **Medero, Richard**
**Lutz, FL 33549 (US)**

• **Hood, Rush W., Jr.**
**Tampa, FL 33624 (US)**

(74) Representative: **Jones, Alan John et al**
**CARPMAELS & RANSFORD**
**43 Bloomsbury Square**
**London, WC1A 2RA (GB)**

(56) References cited:
**EP-A- 0 020 110**        **GB-A- 1 589 391**
**GB-A- 2 092 309**        **US-A- 4 461 266**

Remarks:
The file contains technical information submitted after the application was filed and not included in this specification

## Description

This invention relates to automated blood pressure measuring apparatus and, more particularly to stored program controlled monitors employing the oscillometric method of detection characterised by data purification and enhanced systolic, diastolic and mean blood pressure determination.

Reference is hereby made to the following concurrently filed co-pending European patent applications:

IMPROVED SPHYMOMANOMETRIC CUFF PRESSURISING SYSTEM, EP-A-0207805;

OSCILLOMETRIC BLOOD PRESSURE MONITOR EMPLOYING NON-UNIFORM PRESSURE DECREMENTING STEPS, EP-A-0208520;

IMPROVED AUTOMATED MEAN ARTERIAL BLOOD PRESSURE MONITOR WITH DATA ENHANCEMENT, EP-A-0208521; and

IMPROVED AUTOMATED DIASTOLIC BLOOD PRESSURE MONITOR WITH DATA ENHANCEMENT, EP-A-0207807.

Automated blood pressure monitoring has rapidly become an accepted and, in many cases, essential aspect of human and veterinary treatment. Such monitors are now a conventional part of the patient environment in emergency rooms, intensive and critical care units, and in the operating theatre.

The so-called oscillometric method of measuring blood pressure is one of the most popular methods in commercially available systems. This method relies on measuring changes in arterial counterpressure, such as imposed by an inflatable cuff, which is controllably relaxed or inflated. In some cases the cuff pressure change is continuous, and in others it is incremental. In substantially all, a transducer monitors arterial counterpressure oscillations, and processing apparatus converts select parameters of these oscillations into blood pressure data.

The principles of the present invention are described in US-4360029 and US-4349034, which are commonly assigned with the instant invention. Both patents disclose apparatus and methods for artifact rejection in oscillometric systems. In accordance with the principles described in these patents, an inflatable cuff is suitably located on the limb of a patient, and is pumped up to a predetermined pressure. Thereupon, the cuff pressure is reduced in predetermined fixed decrements, at each level of which pressure fluctuations are monitored. These typically consist of a DC voltage with a small superimposed variational component caused by arterial blood pressure pulsations (referred to herein as "oscillatory complexes"). Therefore, after suitable filtering to reject the DC component and to provide amplification, pulse peak amplitudes above a given threshold are measured and stored. As the decrementing continues, the peak amplitudes will normally increase from a lower amount to a relative maximum, and thereafter will decrease. The lowest cuff pressure at which the oscillations have a maximum peak value is representative of mean arterial pressure. The cuff pressures obtained when stored oscillation complex pulse peak amplitudes bear predetermined fractional relationships with the largest stored peak correspond to the subject's systolic and diastolic pressures.

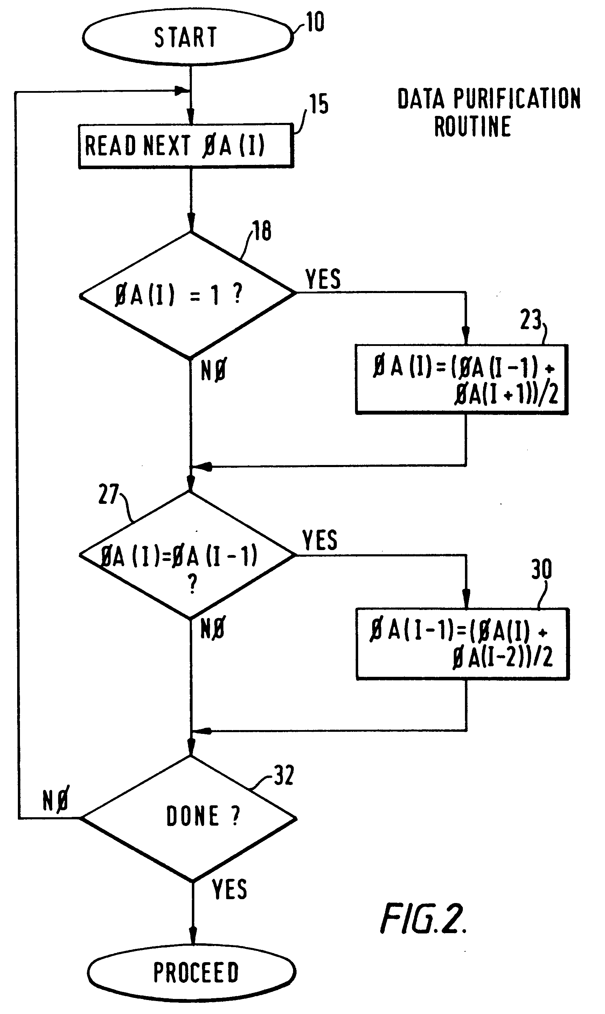

US-4360029 and US-4349034 describe the rejection of artifact data to derive accurate blood pressure data. Indeed, as is apparent from Fig. 2 of both patents, the most substantial portion of the measurement cycle (denominated "T3") is devoted to the execution of complex detection at the various pressure levels, measurement of signal peaks of true complexes, and processing those peaks in accordance with artifact rejection algorithms. Notwithstanding such efforts, the signal peak data collected sometimes incorporates data errors, i.e., a data pattern inconsistent with the above described typical physiological response pattern of a subject as the artery occluding cuff pressure monotonically decreases.

In EP-A-0208520, oscillometric blood pressure measurements are effected with non-uniform, cuff pressure-dependent pressure decrements between successive oscillatory complex peak measuring intervals. Such a method of effecting oscillometric blood pressure measurements is facilitated by systolic, diastolic and mean blood pressure determining algorithms not heretofore employed.

GB-A-1589391 describes an apparatus and process for determining systolic pressure in which a blood pressure cuff is used to apply pressure to the arm of a patient. Values of applied pressure are supplied to an interpolation unit, adjacent measurements being interpolated in order to determine the systolic pressure.

US-A-4461266 discloses a blood pressure monitor which uses incremental steps to obtain blood pressure measurements using the oscillometric method. The pulse amplitudes indicate mean arterial pressure when these start to decrease for increasing cuff pressure. Thus, when pulse amplitudes decrease, a large decrease in cuff pressure is made and the incremental step process is restarted until the maximum pulse amplitude is obtained.

EP-A-0020110 describes a non-invasive vascular waveform transducer and apparatus which can be used to monitor blood flow through the brachial artery when partially occluded by a cuff. Signals which represent the pressure wave proportional to the blood flow and the turbulence created by the blood flow through the partially occluded artery are used to determine heart rate, systolic and diastolic pressures.

GB-A-2092309 describes a technique wherein blood pressure is measured by using an inflatable cuff to apply external pressure to a patient's body member, and measuring the amplitude of pressure oscillation induced in the cuff by blood flow in the body member as the cuff pressure is decreased in steps from a magnitude above an expected systolic (or diastolic) pressure to a magnitude below the expected systolic (or diastolic) blood pressure. The systolic (or diastolic) blood pressure is then determined by making a first linear approximation to a plurality of amplitude measurements occurring below the expected pressure and making a second linear approximation to a plurality of points occurring above the expected pressure, and finding the systolic (or diastolic) pressure by setting the two approximations equal to determine their intercept. Both the systolic and the diastolic blood pressure, and also the mean arterial pressure (where the pressure oscillations reach a maximum) may be measured automatically using a microprocessor.

It is an object of the present invention to provide improved oscillometric blood pressure determining apparatus and methodology.

More specifically, it is an object of the present invention to purify the oscillatory complex peak amplitude data ensemble employed for blood pressure determination.

Yet another object of the present invention is the provision of improved algorithms, methodology and apparatus for determining systolic, diastolic and mean arterial blood pressure.

A blood pressure cuff is applied about a subject's artery, and inflated above the systolic level, thus fully occluding the artery for a full heart cycle. The cuff pressure is thereafter reduced to permit an increasing flow through the progressively less occluded artery, and a measure of the peak amplitudes of the successively encountered oscillatory complexes stored in memory. Also retained is the cuff pressure obtaining for each stored complex peak.

In accordance with varying aspects of the present invention, the stored complex peak-representing data set is corrected for aberrations; and improved data processing operates on the stored (and advantageously corrected) pulse peak data and the corresponding cuff pressure information to determine the subject's systolic, diastolic and mean arterial pressure.

According to the invention, there is provided an automated blood pressure monitor, comprising: an inflatable cuff; means for inflating and deflating said cuff, said means being arranged to deflate said cuff in discrete pressure steps of at least 7 Torr (0.933 KPa); pressure transducer means coupled to said cuff for cuff pressure measurement; means responsive to said cuff pressure measurement for generating a signal representing blood pressure pulses; complex peak storing means for storing values characterising the peak amplitudes of said detected pulses at different cuff pressures; cuff pressure storing means for storing the cuff pressures associated with said cuff pressure pulse peak signals; means for locating the maximum complex peak amplitude stored in said complex peak storing means; means for generating a threshold level which is a predetermined fraction of said maximum peak amplitude value; means for selecting peak amplitudes from said complex peak storing means, the selected amplitudes corresponding to a pair of steps which are prior in time to the time at which the maximum peak amplitude occurred and which respectively precede and follow a cuff pressure associated with said threshold level and being contiguous with the threshold level and generated at cuff pressures higher than that associated with the maximum pulse peak signal; and interpolation means for determining systolic pressure from the selected peak amplitudes contiguous with the threshold level and from the corresponding cuff pressure stored in said cuff pressure storing means, assuming a predetermined functional progression in amplitude between the selected peak amplitudes contiguous with the threshold level (LVL).

According to a further aspect of the present invention, there is provided a method for measuring systolic pressure using an automatic oscillometric blood pressure monitor having a pressurized cuff, means for deflating said cuff, and means for measuring arterial pressure oscillation complexes and the peak of the envelope thereof through measurement of prevailing and time varying cuff pressures, the method comprising the steps of: a) deflating said cuff in discrete pressure steps of at least 7 Torr (0.933 KPa); b) after each said step, detecting oscillation complexes, measuring and storing the peak of the envelope thereof, and storing identification of the associated step; c) finding the amplitude of the largest of said peaks; d) developing a threshold level which is a predetermined fraction of the maximum peak amplitude; e) identifying a pair of steps which are prior in time to the time at which the maximum peak amplitude occurred, and which respectively precede and follow a cuff pressure associated with said threshold level; and f) developing systolic pressure as the interpolated cuff pressure between said pressure levels, assuming a predetermined functional progression in amplitude between the amplitudes and at said pair of steps.

The preferred embodiment of the invention will now be described with reference to the accompanying drawings, in which:

Fig. 1 is a timing diagram illustraing data generation and correction during an illustrative measurement cycle for oscillometric blood pressure determination in accordance with the principles of the present invention;

Fig. 2 is a flow chart illustrating data purification for improved oscillometric blood pressure determination;

Fig. 3 depicts oscillation amplitude processing for a systolic blood pressure measurement in accordance with the present invention;

Fig. 4 is a program flow chart for the systolic blood pressure measurement typified in Fig. 3;

Fig. 5 illustrates blood pressure interpolation for the processing mode of Figs. 3 and 4 (and by analogy for Figs. 6-9 as well);

Fig. 6 depicts oscillatory complex measuring wave forms illustrating diastolic blood pressure determination in accordance with the present invention;

Fig. 7 is a program flow chart illustrating the diastolic blood pressure measurement typified by Fig. 6;

Fig. 8 is a timing diagram depicting oscillatory complex peak amplitude processing for mean arterial pressure measurements in accordance with the present invention; and

Fig. 9 is a program flow chart illustrating the mean arterial pressure determination typified by Fig. 8.

US-4360029, US-4349034, US-4543962, and EP-A-0208520 describe in detail the basic oscillometric method of measuring blood pressure forming a background and a starting point for the instant invention. These patents are incorporated herein by reference.

An artery-occluding cuff is disposed on the subject, e.g., about a subject's upper arm over the brachial artery. At the inception of a measuring cycle, the cuff is inflated to a pressure which fully occludes the brachial artery, i.e., prevents blood from flowing therethrough at any point in the heart cycle. The cuff is then progressively deflated, as in discrete steps. A pressure transducer is coupled to the internal cuff pressure and provides an analog signal characterising the blood pressure oscillatory complexes when they begin to occur (i.e., when the maximum heart pressure corresponding to contraction of the heart's left ventricle exceeds the instantaneously obtaining artery-occluding cuff pressure). The peak values of the complex signals are determined in hardware or software.

As the measurement cycle progresses, the peak amplitude of the blood pressure complexes generally become monotonically larger to a maximum and then become monotonically smaller as the cuff pressure continues toward deflation. The peak amplitude of the cuff pressure oscillation complexes, and the corresponding occluding-cuff pressure values are retained in computer memory. US-4360029, US-4349034, US-4543962 and EP-A-0208520 illustrate previously employed algorithms for processing the stored blood pressure complex peak values and concomitant pressure values to yield the subject's mean arterial pressure. These patents and applications also furnish detailed procedures for measuring oscillatory complex peaks; procedures for testing complexes and rejecting bad data associated with measurement-impeding artifacts (such as motion) during a measurement cycle, and the like.

The oscillometric blood pressure measurements as typified by the disclosed principles are effected under stored program control, as via a microprocessor operative in conjunction with a program containing read only memory (ROM or PROM), and a variable content random access memory (RAM) which stores the cuff pressures, oscillatory complex peak amplitudes, and other processing operand variables. The microprocessor receives the cuff pressure readings generated by the pressure transducer, for example as processed by a peak detector, amplifier and analog-to-digital convertor, and supplies all output control signals required, e.g., to open and close one or more cuff deflating valves.

The cuff may be inflated directly by an air pump; and deflated in fixed, discrete steps under microprocessor control. Alternatively, the cuff may be principally or entirely inflated by the pressurised contents of an air reservoir; and/or deflation may proceed in variable, cuff pressure-dependent steps via selected one or ones of plural deflating valves. These latter alternatives achieve the desideratum of condensing the time required for a composite measurement cycle of operation.

Also, there are alternative procedures for measuring the oscillatory complex peak amplitude at any prevailing cuff pressure. In one mode heretofore employed, plural (e.g., two) complex peaks are measured at each cuff pressure step during cuff deflation, and their average used as the peak value. Since the peaks should be approximately equal, any marked disparity (e.g., > 20%) signals that some artifact error occurred and the data is rejected. In a fast ("stat") mode, after several intervals of qualifying (close or equal peak values) companion complexes are detected to develop measurement confidence, only one pulse is required during succeeding cuff deflation intervals thus speeding the composite measurement period.

As alluded to above, it is sometimes the case when blood pressure complexes are being examined for peak amplitude at any occluding pressure level that improper data is developed. There are varying causes for such aberrations. Perhaps the most common is spurious motion by the subject which generates an inadvertent pressure impulse in the cuff which is sensed by the pressure transducer which may be then incorrectly reflected in the blood pressure measurement. Other causes include varying sources of interfering electrical noise or internal cardiac or respiratory changes in the subject. When a false complex peak amplitude value is generated, it is discarded by the composite measuring apparatus and a discard-signalling value (e.g., +1) retained in its place in memory.

A second form of spurious data occurs when the pattern of stored pulse peak values departs from the physiologically mandated sequence of values which progressively increase to a peak and then progressively decrease.

Attention will now be directed to data processing under stored program control for purifying the data collected by the above-described blood pressure measuring apparatus. Further, specific illustrative efficient algorithms are discussed for in fact determining the subject's systolic. diastolic and mean arterial blood pressures. Such data processing may be effected on any computing equipment, preferably digital microprocessors such as commercially available from a number of vendors. The program instructions and sequences presented below are for illustrative purposes only. Such instructions may in fact be implemented in any of diverse program languages and sequences readily apparent to those skilled in the art. In the signal processing below discussed, processing variables have the following significance:

### I. Variables Employed For All Data Processing Below Discussed

| Variable | Functional Quantity Represented |
|---|---|
| CP(I) | The cuff pressure, measured by the transducer pneumatically coupled to the artery occluding cuff, obtaining during the i-th deflation step. CP(I) is an indexed array, i.e., there exists a plurality of values for CP(I) characterizing each of the $\underline{i}$ deflation steps. |
| $\Phi$A(I) | The peak amplitude of the oscillometric oscillation (i.e., the complex peak amplitude) occurring at the i-th step. Where multiple complexes are measured during each prevailing deflation pressure, $\Phi$A(I) is the average of two (or more) peak amplitudes during the i-th step. $\Phi$A(I) is an indexed array. |
| $\Phi$A(MAX) | The peak value of the array of averaged oscillatory blood pressure complex amplitudes. |
| MAX | The time interval when the peak complex $\Phi$A(MAX) occurred. |

### II. Variables Specific To Systolic Pressure Measurement

| Variable | Functional Quantity Represented |
|---|---|
| LVL | An intermediate processing variable representing a predetermined fraction of $\Phi$A(MAX). |
| SYS | The subject's measured systolic pressure. |

### III. Diastolic Pressure Variables

| Variable | Functional Quantity Represented |
|---|---|
| UDLVL and LDLVL | Intermediate processing variables each representing a different fraction of $\Phi$A(MAX). |
| DIAU, DIAL | Intermediate processing variables representing upper and lower interpolated diastolic pressure computational variables. |
| DIA | The subject's measured diastolic pressure. |

### IV. Mean Arterial Pressure Processing Variables

| Variable | Functional Quantity Represented |
|---|---|
| AMP | The complex pulse peak for the deflation interval following that for which the pressure oscillation amplitude was the maximum. |
| MAPL | An intermediate processing variable employed in the final mean arterial pressure computation. |
| MAP | The subject's mean arterial blood pressure. |

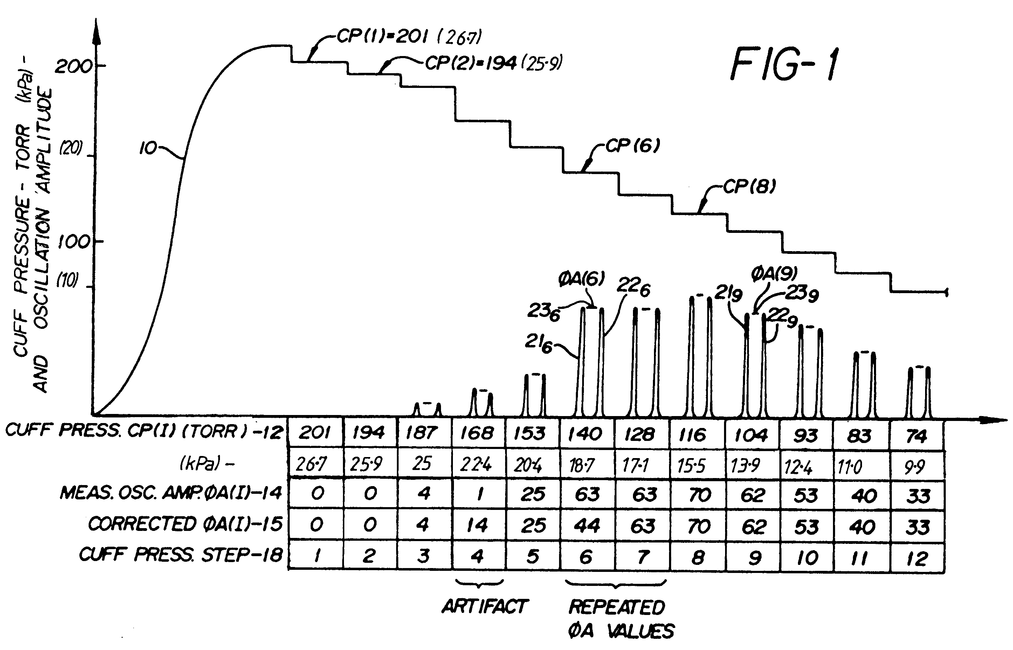

Turning now to Fig. 1, there is depicted wave forms with associated data characterizing the generation of data for an oscillatory blood pressure measurement - and purging (overcoming) bad data constituents. In accordance with the above discussion, the cuff artery occluding pressure for a measurement cycle, as measured by the cuff-associated transducer, is characterized by a wave form 10. The cuff pressure rapidly increases to a maximum above the subject's systolic pressure, and is then deflated in a sequence of steps to a point below the diastolic pressure. The sequence of cuff deflation steps is indicated by the time interval signalling digits 1,2, ... , (lowest row 18 in the data table portion of Fig. 1). The internal pressure characterizing the cuff pressure at each step $\underline{i}$ is given by the data array CP(1),CP(2), ... (upper data table row 12).

Each step (time interval) is made sufficiently long to include at least two heart beats. Accordingly, at least two cuff pressure complex pulses $21_i$ and $22_1$ are measured during each interval after such pulses begin. Legends have been applied to pulses occurring during deflation steps 6 and 9 to avoid clutter and loss of clarity in Fig. 1. No pulses are measured during the first and second pressure steps (time intervals), it being assumed that the cuff pressure [CP(1)=26.7 kPa (201 Torr), and CP(2)=25.9 kPa (194 Torr)] are sufficient during these periods to obviate blood flow through the subject's artery for the full heart cycle. During the following intervals 3,4 ... , two oscillometric complex pulses 21 and 22 are generated and measured, the two pulses having an average peak amplitude 23 (the processor variable array value initially stored in $\Phi A(I)$). The <u>measured</u> oscillation amplitude array ($\Phi A(I)$) is shown in the third row 14 of the Fig. 1 data table for each time interval.

As above noted, assuming a perfect measurement, the oscillation pressure amplitude $\Phi A(I)$ data row would not contain any +1 values which signify an impeded measurement. Further, the data pattern in the third row of the data table for the oscillation amplitudes would exhibit a pattern of successively increasing numbers to a peak value, followed by progressively decreasing values - all without adjacent equal $\Phi A(I)$ values. To the extent that any $\Phi A(I)=1$ values are stored, or to the extent that the progressively increasing/decreasing pattern does not obtain, the data processing in accordance with the instant invention functions to compute appropriate corrected $\Phi A(I)$ values (the third data table row 15 in Fig. 1) for the oscillation amplitude entries requiring correction.

In overview, where any $\Phi A(I)=1$ values exist, they are replaced by the average value of the oscillation amplitude in the two contiguous storage cells, i.e.,

$$\Phi A(I) = (\Phi A(I-1) + \Phi A(I+1))/2. \qquad \text{Eq. 1.}$$

Correspondingly, where two contiguous oscillation amplitudes have the proscribed equal values, the first of the contiguous equal pair is replaced by the average of the amplitudes of the complex peaks measured at the next lower and next higher occluding cuff pressures. See, for example, Eq. 1 and, more particularly, the comparable relationship in functional block 30 of Fig, 2.

Data flow effecting the data purification algorithm above-discussed is set forth in the program flow chart of Fig, 2. Fig. 2 operates on the measured average oscillation amplitudes (the second data table row 14 in Fig. 1) and generates the corrected $\Phi A(I)$ values shown in the third row 15 of Fig. 1. To this end, proceeding from a start block 10 (Fig. 2), step 15 reads the next value $\Phi A(I)$ (proceeding toward the right along the Fig. 1 data table row 14) and test 18 determines whether the value stored in $\Phi A(I)$ equals the error-signalling value +1. If as is the usual case it does not (indicating that the value measured was presumptively free of artifacts and the like), control passes to equality test 27. However, if the contents of $\Phi A(I)$ did equal +1 ("YES" branch of test 18), functional block 23 implements Eq. 1, i.e., replaces the +1 former contents of memory cell $\Phi A(I)$ corresponding to cuff pressure CP(I) with the average value of the oscillation amplitude measured at the next lower ($\Phi A(I-1)$) and next higher non-plus one ($\Phi A(I+1)$) deflation steps. The processing steps 18 and 23 thus purge the measured pressure peak amplitude storage contents (the second row of the Fig. 1 data table) of all +1 values, replacing these by the average value of the measurements made during immediately adjacent deflation steps (corrected $\Phi A(I)$ contents being illustrated in row 15).

Test 27 next examines the current operand $\Phi A(I)$ for the proscribed equality with the previous value $\Phi A(I-1)$. If, as is normally the case, the contents of $\Phi A(I)$ and $\Phi A(I-1)$ differ ("NO" branch from test 27), processing flows to test 32 to determine whether each of the N elements of $\Phi A(I)$ have been processed. If they have not, control returns to block 15 to read in and process the next $\Phi A(I)$ element of the array in the third row 15 of the Fig. 1 data table. When all elements have been processed, control exits from the Fig. 2 data purification routine to data processing point 33 to proceed with the next (unrelated) task for the microprocessor.

If a data error has occurred ("YES" output of test 27 signalling that a data value $\Phi A(I)$ equaled the previous value), control passes to step 30 which replaces the assumed erroneous element $\Phi A(I-1)$ - (the value which should differ from $\Phi A(I)$ but did not) with the average of the two immediately contiguous elements, as by

$$\Phi A(I-1)=(\Phi A(I)+\Phi A(I-2))/2. \qquad \text{Eq. 2.}$$

Accordingly, the data purification routine depicted in Fig. 2 and above-discussed replaces all error reading signifying $\Phi A(I)=1$ values with an interpolated estimated value; and purges the data table row 14 $\Phi A(I)$ array of data of any contiguous equal values. The corrected set of $\Phi A(I)$ is shown in the third row 15 of the Fig. 1 data table. Thus, for example, the oscillation amplitude value during the cuff pressure step (time interval) "4" is corrected from the error-signalling +1 value to a peak amplitude 14, representing the average of measurements 4 and 25 at cuff pressures 25 kPa (187 Torr) and 20.4 kPa (153 Torr) during the immediately contiguous time intervals 3 and 5. Similarly, the first (pressure step 6) of two equal measured oscillation amplitude pulses of value 63 during periods 6 and 7, corresponding to occluding cuff pressures of 18.7 kPa (140 Torr) and 17.1 kPa (128 Torr), is corrected to a value of 44 representing the average of the contiguous measured amplitudes of 63 and 25 units.

The corrected array $\Phi A(I)$ as represented by the fourth row 15 in Fig. 1 thus comprises value from which each of the systolic, diastolic and mean arterial blood pressures may be determined either in accordance with the improved algorithms below discussed or employing the algorithms of the above referenced patents and parent applications. The data purification above discussed provides more accurate measurements than was heretofore the case; and also permits blood pressures to be determined more quickly, obviating the need for repeated deflation steps when unacceptable artifact or noise corrupted data is sensed.

Attention will now be shifted to the particular method pursuant to which the stored cuff pressure CP(I) and corrected blood pressure peak value $\Phi A(I)$ information in the first and fourth data rows of Fig. 1 is employed in accordance with other aspects of the present invention to measure a subject's systolic, diastolic and mean arterial blood pressures.

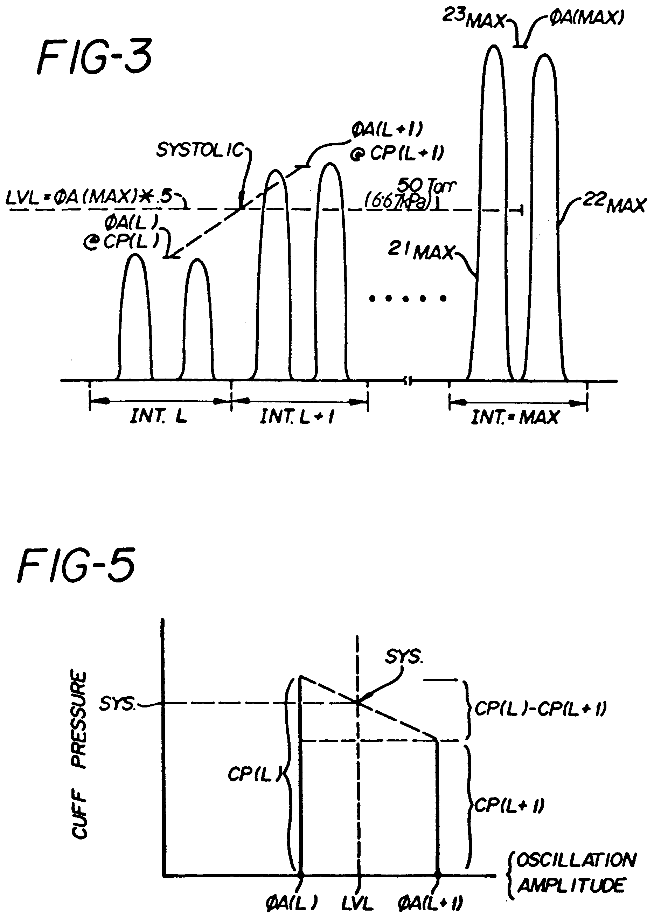

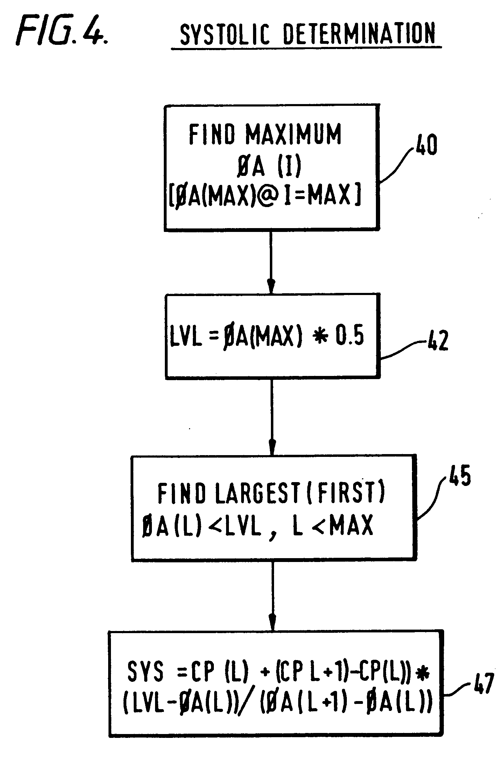

Pulse complex wave form processing typifying systolic blood pressure determination is illustrated in Fig. 3, and a flow chart for the underlying data processing is set forth in Fig. 4. In overview, systolic pressure is determined by:

(a) Finding the amplitude ($\Phi A(MAX)$) of the largest blood pressure oscillatory complex (which occurs at the time interval MAX);

(b) Finding an amplitude level (LVL) equal to a predeterminted fraction of the peak value $\Phi A(MAX)$. We have found a value of 0.5 to be satisfactory for normal processing with something less (e.g., 0.45) for stat (rapid deflation and/or single pulse) operation;

(c) Examining the corrected oscillation amplitude ($\Phi A(I)$) values (third row 15 in the Fig. 1 data table) starting at the MAX interval and proceeding toward the higher cuff pressure direction (i.e., to the left in Figs. 1 and 3) to find the two contiguous oscillation amplitudes for which

$$\Phi A(L) < MAX{*}0.5 < \Phi A(L{+}1); \qquad\qquad \text{Eq. 3.}$$

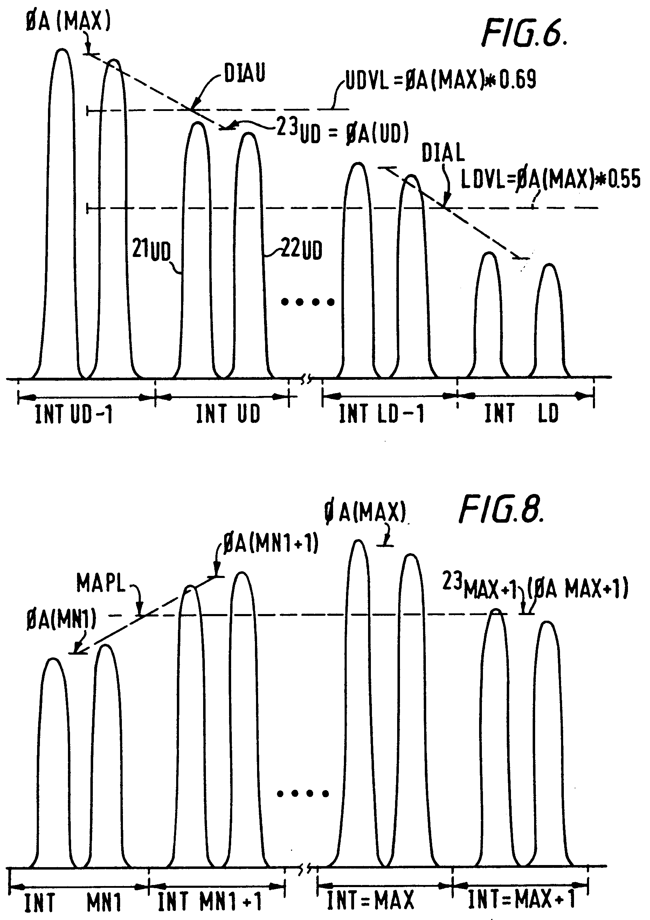

(d) Computing the interpolated cuff pressure (between CP(L) and CP(L+1)) assuming a linear variance in oscillation amplitude and cuff pressure between the intervals L and L+1. This per se well known linear trapezoidal interpolation is graphically depicted in Fig. 5. The interpolated cuff pressure directly corresponds to the subject's systolic blood pressure (SYS). Expanding upon the systolic pressure determining methodology set forth above, the cuff pressure interval I=MAX when the largest oscillation amplitude peak occurs is determined in any per se well known manner, (step 40 of the Fig. 4 flow chart corresponding to the interval MAX in Fig. 3).

Thus, for example, the following schematic BASIC sequence will suffice as illustrative to find the interval MAX:

$$\Phi AMAX=\Phi A(1) \qquad\qquad \text{Eq. 4.}$$

$$MAX=1 \qquad\qquad \text{Eq. 5.}$$

$$FOR\ K=2\ TO\ N \qquad\qquad \text{Eq. 6.}$$

$$IF\ \Phi A(K) < \Phi AMAX\ GOTO\ 70 \qquad\qquad \text{Eq. 7.}$$

$$\Phi AMAX=\Phi A(K) \qquad\qquad \text{Eq. 8.}$$

$$MAX=K \qquad\qquad \text{Eq. 9.}$$

$$70\ NEXT\ K \qquad\qquad \text{Eq. 10.}$$

In brief, Equations 4 and 5 make an initial assumption that the peak value occurred during the first interval and load a provisional peak value storing variable $\Phi AMAX$ with the value $\Phi A(1)$. For an assumed N-time interval measurement, the loop between Equations 6 and 10 sequentially examines every element of the $\Phi A(I)$ array from 2 to N, updating $\Phi AMAX$ only when the value $\Phi A(K)$ - (K being the loop index) exceeds the previously assumed $\Phi AMAX$ value. When the processing exits from the loop following instruction 70 in Equation 10 the variable MAX contains the value of I such that $\Phi A(MAX)$ is the largest value in the array.

The next following step 42 sets a variable LVL equal to the predetermined fraction of the peak amplitude $\Phi A(MAX)$ as by

$$LVL=\Phi A(MAX)^*0.5. \tag{Eq. 11.}$$

The value LVL is shown by the dashed line 50 in Fig. 3.

The next following operation 45 finds the first time interval (L) preceding MAX for which the oscillation amplitude peak is less than LVL, i.e., less than one-half of the peak value $\Phi A(MAX)$, thereby finding the two contiguous values (L, L+1) having peak amplitudes which bound the value in LVL. Algorithms for conducting such a search are well known to those skilled in the art, e.g.,

$$FOR\ J=1\ TO\ MAX \tag{Eq. 12.}$$

$$IF\ (\Phi A(MAX-J)-LVL) < 0\ GOTO\ 140 \tag{Eq. 13.}$$

$$NEXT\ J \tag{Eq. 14.}$$

$$140\ L=MAX-J \tag{Eq. 15.}$$

Equations 12-15 simply-comprise a DO or FOR-NEXT loop progressing from MAX-1 toward L=1, exiting when the first sub-LVL value is obtained. The appropriate interval identification (MAX-J) is stored in the variable location L.

Finally, the value of the systolic pressure is estimated by assuming a linear variation in cuff pressure between the values CP(L) and CP(L+1), and a linear variation between the corresponding oscillation amplitude $\Phi A(L)$ and $\Phi A(L+1)$. Thus, in accordance with the _per se_ well known trapezoidal interpolation equation, the systolic pressure SYS may be determined (step 47 of Fig. 4) by

$$SYS=CP(L) + \frac{(CP)(L+1-CP(L))^*((LVL)-\Phi A(L))}{\Phi A(L+1)-\Phi A(A)} \tag{Eq. 16.}$$

To illustrate employing the data of Fig. 1, 50% of the peak amplitude (9.33) is 4.67, and thus the pulse complex measurements of time intervals 5 and 6 are selected for systolic pressure computation. The Eq. 16 software interpolation implementation yields:

$$SYS= 20.4+((18.67-20.4)x(4.67-3.33)/(5.87-3.33)); \tag{Eq. 17.}$$

$$= 19.5\ kPa \tag{Eq. 18.}$$

assuming three significant figures.

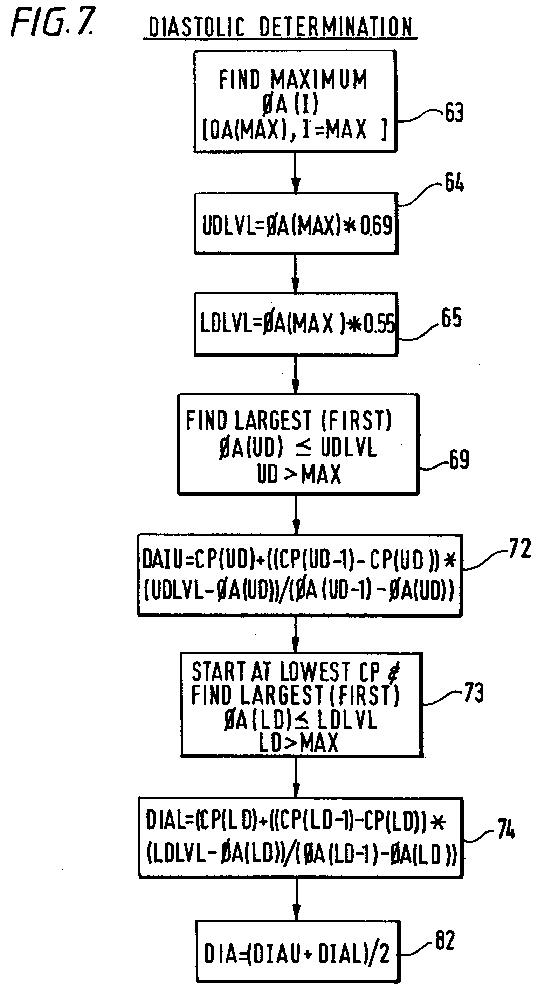

Pulse complex wave form processing characterizing diastolic blood pressure determination is illustrated in Fig. 6; and a flow chart for the underlying diastolic data processing algorithm is depicted in Fig. 7. In overview, diastolic pressure is determined by:

(a) the amplitude ($\Phi A(MAX)$) of the complex (which occurs at the time interval MAX);

(b) Finding an amplitude level (UDLVL) equal to a first predetermined fraction of the peak value $\Phi A(MAX)$. We have found a value of 0.69 to be satisfactory for normal processing and 0.72 for rapid ("stat") processing;

(c) Examining the corrected oscillation amplitude ($\Phi A(I)$) buffer 15 (Fig. 1) starting at the MAX interval and proceeding toward the lower cuff pressure direction (i.e, to the right in Figs. 1 and 6) to find the two contiguous oscillation amplitudes for which

$$\Phi A(UD) \leq MAX^*0.69 \leq \Phi A(UD-1); \tag{Eq. 19.}$$

(d) Finding the interpolated cuff pressure (between CP(UD-1) and CP(UD)) assuming a linear variation in oscillation amplitude and cuff pressure between the intervals UD-1 and UD (processing variable DIAU in Fig. 7);

(e) Examining the stored $\Phi A(I)$ oscillation amplitude values at pressures starting at the lowest cuff pressure measured for a contiguous pair bounding the peak amplitude $\Phi A(MAX)$ multiplied by a second factor lower than the first factor (e.g., 0.55), i.e., where

$$\Phi A(LD) \leq MAX^*0.55 \leq \Phi(LD-1); \tag{Eq. 20.}$$

(f) Computing the interpolated cuff pressure between CP(LD) and CP(LD-1) corresponding to MAX times the 0.55 factor. This lower interpolated cuff pressure is associated with the variable designation DIAL; and

(g) Determining the subject's diastolic pressure (DIA) as the average of the upper and lower interpolated values DIAU and DIAL, i.e.,

$$DIA=(DIAU+DIAL)/2. \qquad \text{Eq. 21.}$$

The above-described procedure is illustrated in the blood pressure complex depiction of Fig. 6 and the Fig. 7 flow chart. The peak $\Phi A(MAX)$ is first located as by the processing of Equations 4-10. The upper and lower peak amplitude fractions DIAU and DIAL are next determined (steps 64 and 65 of Fig. 7 corresponding to the labeled horizontal dash lines in Fig. 6). Step 69 then finds the first time inerval (UD) following MAX at which the peak amplitude $\Phi A(UD)$ is lower than the value stored in DIAU (as by processing analogous to that of Equations 12 through 15 replacing "MAX-J" with "MAX+J"). Thereafter, step 72 performs the trapezoidal interpolation analogous to that of Fig. 5, determining the cuff pressure (DIAU) corresponding to the UDLVL complex amplitude value. It is observed that the time interval UD-1 coincides with the interval MAX when the peak complex value occurred since, for the data case illustrated, the first pulse complex following MAX less than 0.69 x $\Phi A(MAX)$ occurred in the next time interval MAX+1.

The functional steps 73 and 74 of Fig. 7 perform in a manner directly analogous to operations 69 and 72, locating the cuff pressure DIAL by interpolation for the intervals when the peak complex amplitudes bound the LDLVL value equal $\Phi A(MAX)$ times 0.55. This latter search is conducted from $\Phi A(i)$ at the lowest cuff pressure, then working toward higher cuff pressures. Finally, the subject's diastolic pressure (DIA) is computed as the average of the contents stored in DIAU and DIAL (step 82). To illustrate with a numerical example, again employing the data portion of Fig. 1,

$$DIAU= 11.1+((12.4-11.1)x(6.4-5.3))/(5.3-7.1) = 10.3 \text{ kPa} \qquad \text{Eq. 22.}$$

$$DIAL= 9.9+((11.1-9.9)x(5.1-4.4))/(4.4-5.3) = 9 \text{ kPa} \qquad \text{Eq. 23.}$$

$$DIA= (10.3+9)/2 = 9.65 \text{ kPa} \qquad \text{Eq. 24.}$$

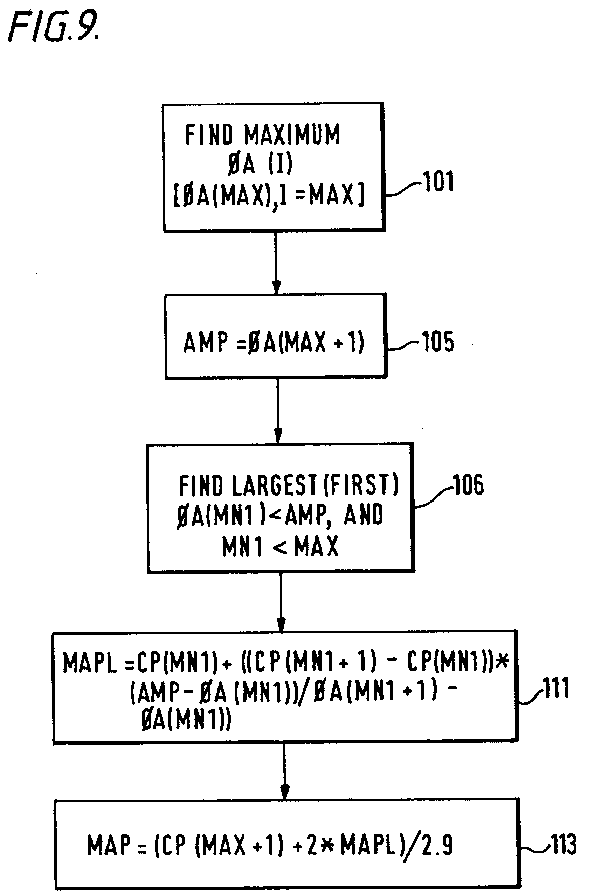

Finally, wave form processing illustrating mean arterial blood pressure measurement is shown in Fig. 8, and in flow chart form for the corresponding data processing in Fig. 9. In summary, mean arterial pressure is determined by:

(a) Finding the amplitude ($\Phi A(MAX)$) of the largest blood complex (which occurs at the time interval MAX);

(b) Examining the cuff pressure values in the corrected register 15 (Fig. 1) for the interval MN1 yielding the first oscillation amplitude less than $\Phi A(MAX+1)$, i.e., the first cuff pressure to the left of the interval MAX which was less than the complex peak amplitude $\Phi A(MAX+1)$ occurring in the first interval following the time MAX. This satisfies the relationship

$$\Phi A(MN1) \leq \Phi A(MAX+1) \leq \Phi A(MN1+1); \qquad \text{Eq. 25.}$$

(c) An interpolation is then conducted between the intervals MN1 and MN1+1 for a cuff pressure MAPL corresponding to the oscillation amplitude value $\Phi A(MAX+1)$; and

(d) Finally, the mean arterial pressure (MAP) is determined by a weighting of the cuff pressures CP(MAX+1) and MAPL, as by

$$MAP=(CP(MAX+1)+(2xMAPL))/2.9 \qquad \text{Eq. 26.}$$

The denominator (2.9 in Eq. 26) may be somewhat lower for operation in a "stat" mode, e.g., 2.85.

The above-discussed algorithm for determining mean arterial pressure is illustrated in Figs. 8 and 9. Step 101 (Fig. 9) finds the peak interval MAX (for example, by execution comparable to Equations 4-10). A processing variable AMP is set equal to the peak value $\Phi A(MAX+1)$ of the complex following the interval MAX (step 105) and the interval MN1 is next determined (step 106) as the first occurring complex less than the value AMP (i.e., $\Phi A(MAX+1)$) to the left of time MAX in Fig. 8 (e.g., by processing comparable to Equations 12-15). An interpolation is then conducted to find the point MAPL (Fig. 8; step 111 in Fig. 9) and the final processing operation 113 finds the subject's mean arterial pressure by implementing Equation 26.

To again illustrate by numerical example from the Fig. 1 data

$$MAPL= 18.7+((17.1-18.7)x(8.3-5.9))/(8.4-5.9) = 17.2 \text{ kPa} \qquad \text{Eq. 27.}$$

$$MAP= (13.9+2x17.2)/2.9 = 16.7 \text{ kPa} \qquad \text{Eq. 28.}$$

The foregoing discussion has thus demonstrated that measured data may be enhanced by replacing data lost through measurement artifacts or the like or deviations from a proper data pattern by approximated values. Specific data processing algorithms were presented and discussed for the computation of a subject's measured systolic, diastolic and mean arterial blood pressures.

The above-described arrangements are merely illustrative of the principles of the present invention. Numerous modifications and adaptations thereof will be readily apparent to those skilled in the art without departing from the scope of the present invention as defined in the present claims. For example, the pressure measurement mode is described above as stepped deflation from an initial inflation above the subject's systolic pressure. The measurement of the instant invention can alternatively be performed by stepped inflation from an initial sub-diastolic cuff pressure.

## Claims

1. An automated blood pressure monitor, comprising:

   an inflatable cuff;

   means for inflating and deflating said cuff, said means being arranged to deflate said cuff in discrete pressure steps of at least 7 Torr (0.933 KPa);

   pressure transducer means coupled to said cuff for cuff pressure (CP(I)) measurement;

   means responsive to said cuff pressure (CP(I)) measurement for generating a signal representing blood pressure pulses;

   complex peak storing means for storing values ($\phi A(I)$) characterising the peak amplitudes of said detected pulses at different cuff pressures (CP(I));

   cuff pressure storing means for storing the cuff pressures (CP(I)) associated with said cuff pressure pulse peak signals ($\phi A(I)$);

   means for locating the maximum complex peak amplitude ($\phi A(MAX)$) stored in said complex peak storing means;

   means for generating a threshold level (LVL) which is a predetermined fraction of said maximum peak amplitude value ($\phi A(MAX)$);

   means for selecting peak amplitudes ($\phi A(I)$) from said complex peak storing means, the selected amplitudes corresponding to a pair of steps (L, L+1) which are prior in time to the time at which ($\phi A(MAX)$) occurred and which respectively precede and follow a cuff pressure associated with said threshold level and being contiguous with the threshold level (LVL) and generated at cuff pressures higher than that associated with the maximum pulse peak signal; and

   interpolation means for determining systolic pressure (SYS) from the selected peak amplitudes ($\phi A(I)$) contiguous with the threshold level (LVL) and from the corresponding cuff pressure (CP(I)) stored in said cuff pressure storing means, assuming a predetermined functional progression in amplitude between the selected peak amplitudes contiguous with the threshold level (LVL).

2. The monitor of claim 1, in which the inflating means inflates the cuff to a predetermined pressure believed to be above systolic pressure (SYS); and further including control means for causing the deflating means to employ deflation decrements at least during a substantial portion of the systolic measurement cycle.

3. The monitor of claim 1 or claim 2, in which the monitor is digital processor controlled and operative in conjunction with a stored program and the means for generating a threshold level (LVL) is a computing means.

4. The monitor of any one of claims 1 to 3, wherein said interpolation means comprises means for computer a pressure intermediate the pressure values retrived from said cuff pressure storing means.

5. The monitor of any one of claims 1 to 4, further including data purifying means operative upon the cuff pressure complex peak amplitude characterizing values ($\phi A(I)$) stored in said complex peak storing means for correcting data inaccuracies.

6. The monitor of claim 5, wherein a preselected character is stored in said complex peak storing means to signal an unsuccessful cuff pressure oscillation peak measurement, and wherein said data purifying means comprises means, responsive to detecting said preselected character, for examining the contents of said complex peak storing means and for replacing said character with a measure of plural stored cuff pressure complex peak values at least one of which was obtained at a cuff pressure higher than that associated with the preselected character and at least one of which was obtained at a cuff pressure lower than that associated with the preselected character.

7. The monitor of claim 6, wherein said examining and replacing means comprises means for replacing said preselected character with the average value of cuff pressure complex peak values stored in said complex peak storing means, at least one of said stored complex peak values having been obtained at a cuff pressure higher than that associated with the preselected character and at least one of which was associated with a cuff pressure lower than that for the preselected character.

8. The monitor of any one of claims 5 to 7, wherein said data purifying means includes means for searching said cuff pressure complex storing means for the occurrence of two equal peak amplitude values arising at successive cuff deflation pressures, and means responsive to said searching means for replacing one of the two stored equal values with the average of the next higher and the next lower values stored in said complex peak storing means.

9. The monitor of claim 8, wherein the replacement measure is the arithmetic average of said two other values.

10. The monitor of claim 9, wherein said replacement measure is the average of the next earlier and next later measured value of the stored complex peak amplitudes.

11. The monitor of any one of claims 5 to 10, in which the data purifying means is operative prior to the means for locating the maximum complex peak amplitude ($\phi$A(MAX)).

12. A method for measuring systolic pressure (SYS) using an automatic oscillometric blood pressure monitor having a pressurized cuff, means for deflating said cuff, and means for measuring arterial pressure oscillation complexes and the peak of the envelope thereof through measurement of prevailing and time varying cuff pressures, the method comprising the steps of:

a) deflating said cuff in discrete pressure steps of at least 7 Torr (0.933 KPa);
b) after each said step, detecting oscillation complexes, measuring and storing the peak ($\phi$A(I)) of the envelope thereof, and storing identification of the associated step;
c) finding the amplitude ($\phi$A(MAX)) of the largest of said peaks;
d) developing a threshold level (LVL) which is a predetermined fraction of the maximum peak amplitude ($\phi$A(MAX));
e) identifying a pair of steps (L, L+1) which are prior in time to the time at which the maximum peak amplitude ($\phi$A(MAX)) occurred, and which respectively precede and follow a cuff pressure associated with said threshold level (LVL); and
f) developing systolic pressure levels (CP(L), CP(L+1)), assuming a predetermined functional progression in amplitude between the amplitudes ($\phi$A(L)) and ($\phi$A(L+1)) at said pair of steps (L, L+1).

13. The method of claim 12, further including, between steps (b) and (c), the step of examining the sequence of said peaks relative to a predetermined overall sequence and substituting an appropriate value for each associated value which departs in predetermined fashion from said sequence.

14. The method of claim 12 or claim 13, wherein said predetermined functional progression is a linear function of time.

**Patentansprüche**

1. Automatisches Blutdrucküberwachungsgerät mit:
   einer aufpumpbaren Manschette ;
   Mittel zum Aufpumpen und zur Druckminderung für die Manschette, wobei mit diesen Mitteln der Manschettendruck in diskreten Druckstufen von zumindest 7 Torr (0,933 KPa) absenkbar ist;
   einem mit der Manschette verbundenen Druckwandler zum Messen des Manschettendrucks (CP(I));
   auf den gemessenen Manschettendruck (CP(I)) ansprechende Mittel zur Erzeugung eines die Blutdruckpulsationen darstellenden Signals;
   eine Speichereinrichtung für die Peakbereiche zur Speicherung von Werten ($\phi$A(I)), die die Spitzenwerte der ermittelten Pulsationen bei verschiedenen Manschettendruckwerten (CP(I)) darstellen;
   eine weitere Speichereinrichtung zur Speicherung der Manschettendruckwerte (CP(I)) in Zusammenhang mit den Spitzensignalen ($\phi$A(I)) der Manschettendruckpulsationen;
   Mittel zur Bestimmung des größten Spitzenwertes ($\phi$A(MAX)) der Peakbereiche, der in der Speichereinrichtung für die Peakbereiche gespeichert ist;

Mittel zur Erzeugung eines Schwellenwertes (LVL), der ein vorgegebener Bruchteil des größten Spitzenwertes (φA(MAX)) ist;

Mittel zur Auswahl von Spitzenwerten (φA(I)) aus der Speichereinrichtung für die Peakbereiche, wobei die ausgewählten Werte einem Stufenpaar (L,L+1) entsprechen, welches dem größten Spitzenwert (φA(MAX)) zeitlich vorangeht, und welches einem dem genannten Schwellenwert zugeordneten Manschettendruck vorangeht bzw. folgt, und wobei die ausgewählten Spitzenwerte benachbart sind zum Schwellenwert (LVL) und bei höheren Manschettendruckwerten erzeugt worden sind als derjenige, der dem maximalen Signal der Pulsationspeake zugeordnet ist; und

Mittel zur Bestimmung des systolischen Blutdrucks (SYS) mittels Interpolation aus den ausgewählten Spitzenwerten (φA(I)) benachbart zum Schwellenwert (LVL) und aus den entsprechenden Manschettendruckwerten (CP(I)) die in dem Speicher für die Manschettendruckwerte abgespeichert sind, wobei eine vorbestimmte funktionale Zunahme zwischen den ausgewählten Spitzenwerten benachbart zum Schwellenwert (LVL) vorausgesetzt ist.

2. Überwachungsgerät nach Anspruch 1, dadurch gekennzeichnet, daß die Mittel zum Aufpumpen der Manschette diese bis zu einem vorgegebenen Druck oberhalb des systolischen Blutdrucks (SYS) aufpumpen; wobei außerdem eine Kontrolleinrichtung vorgesehen ist, die auf die Mittel zur Manschettendruckminderung einwirkt, um zumindest während des Hauptteils des systolischen Meßzyklus den Druck schrittweise zu vermindern.

3. Überwachungsgerät nach Anspruch 1 oder 2, dadurch gekennzeichnet, daß das Überwachungsgerät von einem digitalen Prozessor gesteuert ist und mittels eines gespeicherten Programms arbeitet, und daß die Mittel zur Erzeugung eines Schwellenwertes (LVL) einen Rechner darstellen.

4. Überwachungsgerät nach einem der Ansprüche 1 bis 3, dadurch gekennzeichnet, daß die Mittel zur Interpolation Mittel zur Berechnung eines Druckwertes aufweisen, der zwischen den Druckwerten liegt, die aus der Speichereinrichtung für die Manschettendruckwerte stammen.

5. Überwachungsgerät nach einem der Ansprüche 1 bis 4, dadurch gekennzeichnet, daß eine Datenkorrektureinrichtung vorgesehen ist, welche anhand der die Spitzenwerte in einem Manschettendruckbereich beschreibenden Werte (φA(I)) arbeitet, die in der Speichereinrichtung für die Peakbereiche abgelegt sind, um Datenungenauigkeiten zu korrigieren.

6. Überwachungsgerät nach Anspruch 5, dadurch gekennzeichnet, daß ein vorbestimmtes Zeichen in der Speichereinrichtung für die Peakbereiche gespeichert ist, um eine verfehlte Messung eines Oszillationspeaks des Manschettendrucks anzuzeigen, und daß die Datenkorrektureinrichtung Mittel aufweist, die auf das Auftreten dieses vorgewählten Zeichens ansprechen, um die Inhalte der Speichereinrichtung für die Peakbereiche zu überprüfen, und um das genannte Zeichen durch eine Reihe von gespeicherten Meßwerten aus einem Peakbereich des Manschettendrucks zu ersetzen, wobei zumindest einer dieser Meßwerte bei einem höheren Manschettendruck als der dem vorgewählten Zeichen zugeordnete Druck gewonnen wurde, und zumindest ein anderer Meßwert bei einem tieferen Druck.

7. Überwachungsgerät nach Anspruch 6, dadurch gekennzeichnet, daß die Mittel zur Datenüberprüfung und zum Datenaustausch ihrerseits Mittel zum Ersetzen des vorgewählten Zeichens durch einen Durchschnittswert der Spitzenwerte aus einem Manschettendruckwertebereich, die in der betreffenden Speichereinrichtung gespeichert sind, aufweisen, wobei zumindest einer dieser Spitzenwerte bei einem höheren Manschettendruck als der dem vorgewählten Zeichen zugeordneten Druck erhalten wurde, und zumindest ein anderer dieser Werte einem Manschettendruck zugeordnet war, der niedriger als der für das vorgewählte Zeichen ist.

8. Überwachungsgerät nach einem der Ansprüche 5 bis 7, dadurch gekennzeichnet, daß die Datenkorrektureinrichtung Mittel aufweist, um die Speichereinrichtung für den Manschettendruckbereich nach dem Auftreten zweier gleich großer Spitzenwerte zu durchsuchen, die bei aufeinanderfolgenden abnehmenden Manschettendruckwerten auftreten, und weiterhin eine auf die genannten Mittel ansprechende Einrichtung, um eine der beiden gespeicherten, gleich großen Werte durch den Mittelwert des nächsthöheren und nächstniedrigeren Wertes zu ersetzen, die in der betreffenden Speichereinrichtung gespeichert sind.

9. Überwachungsgerät nach Anspruch 8, dadurch gekennzeichnet, daß der Ersatzwert das arithmetische Mittel der genannten zwei Werte darstellt.

**10.** Überwachungsgerät nach Anspruch 9, dadurch gekennzeichnet, daß der Ersatzwert der Durchschnittswert des zeitlich vorangehend und des zeitlich nachfolgend gemessenen Wertes der gespeicherten Spitzenwerte im Peakbereich darstellt.

**11.** Überwachungsgerät nach einem der Ansprüche 5 bis 10, dadurch gekennzeichnet, daß die Datenkorrektureinrichtung vor den Mitteln zur Bestimmung der des größten Spitzenwertes im Peakbereich ($\phi$A(MAX)) wirksam ist.

**12.** Verfahren zur Messung des systolischen Blutdrucks (SYS) unter Verwendung eines automatischen oszillatorischen Blutdruckmeßgeräts mit einer druckbeaufschlagten Manschette, Mittel zur Manschettendruckminderung und einer Einrichtung zur Messung des arteriellen Blutdruckes in Oszillationsbereichen sowie des Maximalwertes seiner Einhüllenden mittels Messung der vorherrschenden und sich zeitlich ändernden Manschettendruckwerte, gekennzeichnet durch folgende Verfahrensschritte:

a) der Manschettendruck wird in diskreten Schritten von zumindest 7 Torr (0,933 KPa) abgesenkt;
b) nach jedem dieser Schritte werden die Oszillationsbereiche ermittelt und der jeweilige Spitzenwert ($\phi$A(I)) ihrer Einhüllenden wird gemessen und gespeichert, wobei eine Kennung des zugehörigen Schrittes mitabgespeichert wird;
c) die Größe ($\phi$A(MAX)) des höchsten dieser Spitzenwerte wird ermittelt;
d) ein Schwellenwert (LVL), der ein vorgegebener Bruchteil dieses größten Spitzenwertes ($\phi$A(MAX)) ist, wird festgelegt;
e) ein Stufenpaar (L, L+1), welches zeitlich dem größten Spitzenwert ($\phi$A(MAX)) vorausgeht, und welches dem dem genannten Schwellenwert (LVL) zugeordneten Manschettendruck vorausgeht bzw. folgt, wird bestimmt; und schließlich
f) werden Größen für den systolischen Blutdruck (CP(L), CP(L+1)) berechnet, wobei eine vorgegebene funktionale Zunahme zwischen den Werten ($\phi$A(L)) und ($\phi$A(L+1)) bei diesem Stufenpaar (L, L+1) vorausgesetzt wird.

**13.** Verfahren nach Anspruch 12, dadurch gekennzeichnet, daß zwischen den Verfahrensschritten (b) und (c) ein weiterer Verfahrenschritt eingefügt wird, bei dem die Folge der genannten Spitzenwerte in bezug zu einer vorherbestimmten Gesamtsequenz untersucht wird und bestimmte Werte durch geeignete Werte ersetzt werden, wenn erstere in vorherbestimmter Weise von denen der genannten Folge abweichen.

**14.** Verfahren nach Anspruch 12 oder 13, dadurch gekennzeichnet, daß die vorherbestimmte funktionale Zunahme eine lineare Funktion der Zeit ist.

## Revendications

**1.** Un appareil automatisé de surveillance de la pression sanguine, qui comprend:

un fourreau gonflable;

un moyen de gonflage et de dégonflage dudit fourreau, ledit moyen étant agencé de manière à dégonfler ledit fourreau par étapes discrètes de pression d'au moins 0,933 KPa (7 Torr);

un moyen transducteur de pression couplé audit fourreau en vue de mesurer la pression (CP(I)) du fourreau;

un moyen qui répond à ladite mesure de pression (CP(I)) du fourreau pour engendrer un signal représentant des impulsions de pression sanguine;

un moyen de mémorisation de pointes complexes pour mémoriser des valeurs ($\Phi$A(I)) qui caractérisent les amplitudes de pointe desdites impulsions détectées à différentes valeurs de pression (CP(I)) du fourreau;

un moyen de mémorisation de pression du fourreau pour mémoriser les pressions (CP(I)) du fourreau associées auxdits signaux ($\Phi$A(I) de pointes d'impulsions de pression du fourreau;

un moyen de localisation de l'amplitude maximale ($\Phi$A(MAX)) de pointe complexe mémorisée dans ledit moyen de mémorisation de pointes complexes.

un moyen de génération d'un niveau de seuil (LVL) qui est une fraction prédéterminée de la dite valeur d'amplitude maximale ($\Phi$A(MAX)) de pointe;

un moyen de sélection d'amplitudes ($\Phi$A(I)) de pointes dans ledit moyen de mémorisation de pointes complexes, les amplitudes sélectionnées correspondant à un paire d'étapes (L, L+1) qui précèdent dans le temps l'instant auquel ($\Phi$A(MAX)) s'est produite et qui précèdent et suivent respectivement une pression de fourreau associée audit niveau de seuil et qui sont contiguës au niveau de seuil (LVL) et engendrées à des pressions de fourreau supérieures à celles qui sont associées au signal maximal de pointe d'impulsion; et

un moyen d'interpolation destiné à déterminer une pression systolique (SYS) à partir des amplitudes sélectionnées ($\Phi$A(I)) de pointes contiguës au niveau de seuil (LVL) et à partir de la pression correspondante (CP(I)) mémorisée dans ledit moyen de mémorisation de pression de fourreau, en supposant une progression fonctionnelle prédéterminée en amplitude entre les amplitudes sélectionnées de pointes contiguës au niveau de seuil (LVL).

2. L'appareil de surveillance selon la revendication 1, dans lequel le moyen de gonflage gonfle le fourreau à une pression prédéterminée que l'on pense supérieure à la pression systolique (SYS); et qui inclut en outre un moyen de commande destiné à amener le moyen de dégonflage à employer des décréments de dégonflage au moins pendant une partie sensible du cycle de mesure systolique.

3. L'appareil de surveillance selon la revendication 1 ou la revendication 2, dans lequel l'appareil de surveillance est commandé par un processeur numérique et peut agir en liaison avec un programme mémorisé, et le moyen de génération d'un niveau de seuil (LVL) est un moyen de calcul.

4. L'appareil de surveillance selon l'une quelconque des revendications 1 à 3, dans lequel ledit moyen d'interpolation comprend un moyen de calcul d'une pression intermédiaire entre les valeurs de pressions restituées par ledit moyen de mémorisation de pression de fourreau.

5. L'appareil de surveillance selon l'une quelconque des revendications 1 à 4, qui inclut en outre un moyen de contrôle des données avant traitement, ou purification des données, qui peut agir sur les valeurs caractérisantes ($\Phi A(I)$) d'amplitude de pointes complexes de pression de fourreau mémorisées dans ledit moyen de mémorisation de pointes complexes pour corriger des inexactitudes de données.

6. L'appareil de surveillance selon la revendication 5, dans lequel un caractère sélectionné au préalable est mémorisé dans ledit moyen de mémorisation de pointes complexes de manière à signaler une mesure sans succès de pointe d'oscillation de pression de fourreau, et dans lequel ledit moyen de contrôle des données avant traitement comprend un moyen, qui répond à une détection dudit caractère sélectionné au préalable, destiné à examiner le contenu dudit moyen de mémorisation de pointes complexes et à remplacer ledit caractère par une mesure de plusieurs valeurs mémorisées de pointes complexes de pression de fourreau dont au moins une a été obtenue à une pression de fourreau supérieure à celle qui est associée au caractère sélectionné au préalable et au moins une a été obtenue à une pression de fourreau inférieure à celle qui est associée au caractère sélectionné au préalable.

7. L'appareil de surveillance selon la revendication 6, dans lequel ledit moyen d'examen et de remplacement comprend un moyen de remplacement dudit caractère sélectionné au préalable par la valeur moyenne de valeurs de pointes complexes de pression de fourreau mémorisées dans ledit moyen de mémorisation de pointes complexes, parmi lesquelles au moins l'une desdites valeurs mémorisées de pointes complexes a été obtenue à une pression de fourreau supérieure à celle qui est associée au caractère sélectionné au préalable et au moins une a été associée à une pression de fourreau inférieure à celle qui concerne le caractère sélectionné au préalable.

8. L'appareil de surveillance selon l'une quelconque des revendications 5 à 7 dans lequel ledit moyen de contrôle des données avant traitement inclut un moyen d'exploration dudit moyen de mémorisation de pointes complexes de pression de fourreau quant à l'apparition de deux valeurs égales d'amplitude de pointes se produisant à des pressions successives de dégonflage du fourreau, et un moyen qui répond audit moyen d'exploration pour remplacer l'une des deux valeurs égales mémorisées par la moyenne des deux valeurs immédiatement supérieures ou immédiatement inférieures mémorisées dans ledit moyen de mémorisation de pointes complexes.

9. L'appareil de surveillance selon la revendication 8, dans lequel la valeur de remplacement est la moyenne arithmétique desdites deux autres valeurs.

10. L'appareil de surveillance selon la revendication 9, dans lequel la valeur de remplacement est la moyenne des valeurs mesurées, immédiatement précédente et immédiatement suivante, des amplitudes mémorisées de pointes complexes.

11. L'appareil de surveillance selon l'une quelconque des revendications 5 à 10 dans lequel le moyen de contrôle des données avant traitement agit avant le moyen de localisation de l'amplitude maximale ($\Phi A(MAX)$) des pointes complexes.

12. Un procédé de mesure de la pression systolique (SYS) qui utilise un appareil oscillométrique automatique de surveillance de la pression sanguine qui comporte un fourreau sous pression, un moyen de dégonflage dudit fourreau, et un moyen destiné à mesurer des valeurs complexes d'oscillation de la pression artérielle et la pointe de leur enveloppe en mesurant des pressions de fourreau existantes et variables dans le temps, le procédé comprenant les étapes consistant à:

a) dégonfler ledit fourreau par étapes discrètes d'au moins 0,933 KPa (7 Torr);

b) après chaque étape, détecter des valeurs complexes d'oscillation, mesurer et mémoriser la pointe ($\Phi$A(I)) de leur enveloppe, et mémoriser une identification de l'étape associée;

c) trouver l'amplitude maximale ($\Phi$A(MAX)) de la plus grande desdites pointes;

d) calculer un niveau de seuil (LVL) qui est une fraction prédéterminée de la dite amplitude maximale ($\Phi$A(MAX)) de pointe;

e) identifier un paire d'étapes (L, L+1) qui précèdent dans le temps l'instant auquel l'amplitude maximale ($\Phi$A(MAX)) de pointe s'est produite et qui précèdent et suivent respectivement une pression de fourreau associée audit niveau de seuil ((LVL); et

f) calculer des niveaux (CP(L)), (CP(L+1)) de pression systolique (SYS) en supposant une progression fonctionnelle prédéterminée en amplitude entre les amplitudes ($\Phi$A(L)) et ($\Phi$A(L + 1)) à ladite paire d'étapes (L, L + 1).

13. Le procédé selon la revendication 12, qui inclut en outre, entre les étapes (b) et (c), l'étape consistant à examiner la séquence desdites pointes par rapport à une séquence d'ensemble prédéterminée et à substituer une valeur appropriée à chaque valeur associée qui s'écarte de ladite séquence d'un façon prédéterminée.

14. Le procédé selon la revendication 12 ou la revendication 13, dans lequel ladite progression fonctionnelle prédéterminée est une fonction linéaire du temps.

**FIG-1**

CUFF PRESSURE - TORR (kPa) - AND OSCILLATION AMPLITUDE

CP(I)=201 (26·7)
CP(2)=194 (25·9)
CP(6)
CP(8)

ØA(6) 22·6
23·6
21·6
21·9 ØA(9) 23·9
22·9

10

| CUFF PRESS. CP(I) (TORR) -12 | 201 | 194 | 187 | 168 | 153 | 140 | 128 | 116 | 104 | 93 | 83 | 74 |
|---|---|---|---|---|---|---|---|---|---|---|---|---|
| (kPa) – | 26·7 | 25·9 | 25 | 22·4 | 20·4 | 18·7 | 17·1 | 15·5 | 13·9 | 12·4 | 11·0 | 9·9 |
| MEAS. OSC. AMP. ØA(I)–14 | 0 | 0 | 4 | 1 | 25 | 63 | 63 | 70 | 62 | 53 | 40 | 33 |
| CORRECTED ØA(I)–15 | 0 | 0 | 4 | 14 | 25 | 44 | 63 | 70 | 62 | 53 | 40 | 33 |
| CUFF PRESS. STEP–18 | 1 | 2 | 3 | 4 | 5 | 6 | 7 | 8 | 9 | 10 | 11 | 12 |

ARTIFACT          REPEATED ØA VALUES

EP 0 207 806 B1

FIG.2.

## FIG-3

## FIG-5

## FIG. 4.    SYSTOLIC DETERMINATION

FIND MAXIMUM
$\emptyset A$ (I)
[$\emptyset A$(MAX)@ I=MAX]    — 40

$LVL = \emptyset A(MAX) * 0.5$    — 42

FIND LARGEST (FIRST)
$\emptyset A(L) < LVL$, $L < MAX$    — 45

$SYS = CP(L) + (CP \; L+1) - CP(L)) *$
$(LVL - \emptyset A(L)) / (\emptyset A(L+1) - \emptyset A(L))$    — 47

FIG.6.

FIG.8.

*FIG.7.*     <u>DIASTOLIC DETERMINATION</u>

FIND MAXIMUM
$\emptyset A (I)$
$[OA(MAX), I=MAX]$ — 63

$UDLVL = \emptyset A(MAX) * 0.69$ — 64

$LDLVL = \emptyset A(MAX) * 0.55$ — 65

FIND LARGEST (FIRST)
$\emptyset A(UD) \leq UDLVL$
$UD > MAX$ — 69

$DAIU = CP(UD) + ((CP(UD-1) - CP(UD)) *$
$(UDLVL - \emptyset A(UD))/(\emptyset A(UD-1) - \emptyset A(UD))$ — 72

START AT LOWEST CP $\not c$
FIND LARGEST (FIRST)
$\emptyset A(LD) \leq LDLVL$
$LD > MAX$ — 73

$DIAL = (CP(LD) + ((CP(LD-1) - CP(LD)) *$
$(LDLVL - \emptyset A(LD))/(\emptyset A(LD-1) - \emptyset A(LD))$ — 74

$DIA = (DIAU + DIAL)/2$ — 82

*FIG.9.*

$$\boxed{\begin{array}{c} \text{FIND MAXIMUM} \\ \emptyset A \ (I) \\ [\emptyset A(MAX), I = MAX] \end{array}} \sim 101$$

$$\boxed{AMP = \emptyset A(MAX + 1)} \sim 105$$

$$\boxed{\begin{array}{c} \text{FIND LARGEST (FIRST)} \\ \emptyset A(MN1) < AMP, \text{ AND} \\ MN1 < MAX \end{array}} \sim 106$$

$$\boxed{\begin{array}{c} MAPL = CP(MN1) + ((CP(MN1+1) - CP(MN1)) * \\ (AMP - \emptyset A(MN1)) / \emptyset A(MN1+1) - \\ \emptyset A(MN1)) \end{array}} \sim 111$$

$$\boxed{MAP = (CP(MAX+1) + 2 * MAPL)/2.9} \sim 113$$